# EUROPÄISCHE PATENTSCHRIFT

(11) **EP 2 437 839 B1**
(45) Veröffentlichungstag und Bekanntmachung des Hinweises auf die Patenterteilung: **17.04.2013**
(21) Anmeldenummer: 10725603.4
(22) Anmeldetag: 04.06.2010
(51) Int. Cl.: A61M 21/00, A61N 5/06

(54) **LICHTBEHANDLUNGSVORRICHTUNG**
LIGHT TREATMENT APPARATUS
DISPOSITIF DE PHOTOTHÉRAPIE

(30) Priorität: 05.06.2009 DE 202009007912 U
(43) Veröffentlichungstag der Anmeldung: 11.04.2012
(73) Patentinhaber: Winkler, Engelbert, 6300 Wörgl (AT); Proeckl, Dirk, 6300 Wörgl (AT)
(72) Erfinder: Winkler, Engelbert, 6300 Wörgl (AT); Proeckl, Dirk, 6300 Wörgl (AT)
(74) Vertreter: Thoma, Michael
(86) Internationale Anmeldenummer: PCT/EP2010/003381
(87) Internationale Veröffentlichungsnummer: WO 2010/139480

(56) Entgegenhaltungen:
- US-A- 5 403 261
- US-A1- 2006 047 324
- US-A1- 2006 106 276

## Beschreibung

Die vorliegende Erfindung betrifft eine Lichtbehandlungsvorrichtung zur Simulation einer psychophysischen Grenzerfahrung, mit einer Lichtabgabevorrichtung zur Abgabe von über das menschliche Auge aufnehmbarem Licht, sowie einer Steuervorrichtung zur Steuerung der Lichtabgabevorrichtung.

Es ist bekannt, dass extreme psychophysische Bedingungen körperliche und geistige Regenerationsprozesse auszulösen vermögen, die zu einer Neuausrichtung des gesamten Organismus führen und Gesundheit unmittelbar erfahrbar machen. Derartige psychophysische Extrembedingungen treten beispielsweise während einer Nahtoderfahrung, der sog. "Near Death Experience" auf, aber auch im Hochleistungssport oder in tiefer Meditation. Betroffene Personen, die eine solche Grenzerfahrung bzw. eine sog. "Peak Experience" machen, berichten regelmäßig von einer Beschleunigungs- bzw. Tunnelerfahrung mit einem besonders hellen Licht am Ausgang.

Einhergehend mit einer solchen psychophysischen Grenzerfahrung sind auf verschiedenen Ebenen spürbare und messbare Therapieeffekte. Einerseits können auf psychologischer Ebene die Therapieeffekte von einer deutlich spürbaren Stimmungsaufhellung, über ein starkes Wohlbefinden bis zu einer tiefenpsychologischen Entspannung reichen. Andererseits sind physiologisch konkrete Veränderungen messbar, die beispielsweise im EEG sichtbar werden oder sich in veränderten Blutwerten niederschlagen.

Bisherige Lichtbehandlungsgeräte werden zu verschiedenen therapeutischen Zwecken eingesetzt. So ist es beispielsweise bekannt, den Serotoninspiegel durch Lichtbestrahlung eines Probanden zu verändern, um Schlafstörungen zu beseitigen, Niedergeschlagenheit zu mildern und Depressionen abzubauen. Ein entsprechendes Lichtbehandlungsgerät ist beispielsweise unter dem Handelsnamen "Davita Lichtdusche Physiolight LD 220" bekannt, oder auch in ähnlicher Form in der DE 20 2005 010 124 U1 beschrieben. Andererseits werden beispielsweise Tageslichtlampen zur Bestrahlung von Probanden eingesetzt, um winterlichen Lichtmangel auszugleichen und Winterdepressionen zu mindern.

Derartige Geräte sind indes allesamt nicht geeignet, psychophysische Grenzerfahrungen der genannten Art herbeizuführen, die tiefenpsychologische Therapieeffekte mit physischen Therapieeffekten in der genannten Art verbinden.

US-2006/106276 beschreigt eine Lichtbehandlungsvorrichtung gemäss dem Oberbegriff des Anspruchs 1.

Der Erfindung liegt daher die Aufgabe zugrunde, ein verbessertes Lichtbehandlungsgerät zur Simulation von psychophysischen Grenzerfahrungen zu schaffen, das Nachteile des Standes der Technik vermeidet und Letzteren in vorteilhafter Weise weiterbildet. Insbesondere soll mit einem einfachen Geräteaufbau eine intensive Simulation von psychophysischen Grenzerfahrungen auch bei nur kurzen Behandlungssitzungen erzielt werden.

Erfindungsgemäß wird diese Aufgabe durch ein Lichtbehandlungsgerät gemäß Anspruch 1 gelöst. Bevorzugte Ausgestaltungen der Erfindung sind Gegenstand der abhängigen Ansprüche.

Es wird also vorgeschlagen, auf verschiedenen Wahrnehrnungsstufen wirkendes Licht gleichermaßen zu erzeugen und einen Probanden damit zu beaufschlagen. Einerseits wird ein Permanentlicht bzw. Konstantlicht, das sich zwar kontinuierlich in seiner Stärke oder Lichtfarbe ändern kann, jedoch keine Aussetzer hat, erzeugt, während andererseits ein Flackerlicht erzeugt wird, das dem genannten Dauerlicht überlagert werden, so dass ein Proband mit beiden Lichtdosen gleichzeitig beaufschlagt wird, wobei das Flackern des Flackerlichts kontinuierlich oder stufenweise beschleunigt wird, um einen starken Beschleunigungseffekt zu induzieren. Erfindungsgemäß besitzt die Lichtabgabevorrichtung zumindest eine Dauerlichtquelle sowie zumindest eine Flackerlichtquelle, deren Flackerlicht dem Dauerlicht der Dauerlichtquelle im Bereich eines Therapieplatzes überlagerbar ist, umfasst, wobei die Steuervorrichtung einen Frequenzsteuerbaustein aufweist, der in zumindest einem Beschleunigungsdurchlauf die Frequenz der Flackerlichtquelle von einer Ausgangsfrequenz auf eine Zielfrequenz erhöht oder vermindert. Durch die kombinatorische Wirkung der beiden Lichtquellen kann eine psychophysische Grenzerfahrung simuliert werden, die intensive Therapieeffekte sowohl auf psychologischer Ebene als auch auf physischer Ebene herbeiführt. Insbesondere kann die Frequenz der Flackerlichtquelle in dem Beschleunigungsdurchlauf auf eine Zielfrequenz erhöht werden, die zumindest doppelt so hoch ist wie die Ausgangsfrequenz. Während hierdurch die Beschleunigung der Flackerlichtfrequenz einen starken Beschleunigungseffekt beim Probanden induziert, simuliert das Konstant- bzw. Permanentlicht die eigentliche sog. Lichterfahrung der Todesnähe bzw. die eingangs genannte Tunnelerfahrung mit einem besonders hellen Licht am Ausgang. Hierdurch wird eine starke psychophysisch spürbare Transzendenzerfahrung erzielt, die körperliche Schmerzen oder andere Symptome zu therapieren hilft, aber auch nur zur (tiefen-)psychologischen Entspannung oder im Wellnessbereich eingesetzt werden kann.

In Weiterbildung der Erfindung ist dabei vorgesehen, dass die Steuervorrichtung die Frequenz der Flackerlichtquelle bis zumindest in den Bereich der optischen Fusionsgrenze hochfährt, vorzugsweise über diese hinausfährt, um eine besonders starke Beschleunigungswirkung am Probanden zu induzieren. Diese Beschleunigungswirkung kann hierbei in besonderer Weise dadurch erzielt werden, dass die Frequenz der Flackerlichtquelle über eine ausreichend große Frequenzspanne variiert wird, wobei die Größe der Frequenzvariation an die Dauer des Beschleunigungsdurchlaufs und/oder die Zeitspanne, die für das Hochfahren der Flackerlichtfrequenz von der genannten Anfangsfrequenz bis zur Zielfrequenz benötigt wird, angepasst wird.

In Weiterbildung der Erfindung beträgt die Zielfrequenz mindestens das Fünffache, vorzugsweise mehr als das Zehnfache der Ausgangsfrequenz. Gemäß einer vorteilhaften Ausführung der Erfindung kann der genannte Frequenzsteuerbaustein die Flackerlichtfrequenz im Bereich von 0,1 Hz bis 10000 Hz, vorzugsweise 1 Hz bis 1000 Hz, und nach einer vorteilhaften Ausführung von 2 Hz bis 150 Hz variieren, wobei ggf. bereits eine Variation über einen Teilausschnitt dieses genannten Frequenzbereichs ausreichend sein kann, beispielsweise die Variation der Flackerlichtfrequenz von einer Ausgangsfrequenz von beispielsweise 5 Hz auf eine Zielfrequenz von beispielsweise 50 Hz. In vorteilhafter Weiterbildung der Erfindung ist der Frequenzsteuerbaustein jedoch derart ausgebildet, dass die Flackerlichtfrequenz über den gesamten genannten Bereich variierbar ist.

In vorteilhafter Weiterbildung der Erfindung besitzt der Frequenzsteuerbaustein hierbei Einstellmittel zur variablen Einstellung der Anfangsfrequenz und/oder zur variablen Einstellung der Zielfrequenz, wobei vorteilhafterweise die Anfangsfrequenz beliebig in dem genannten Variationsbereich, vorzugsweise zumindest in dessen unteren Hälfte, und die Zielfrequenz ebenfalls über den gesamten genannten Variationsbereich, vorzugsweise zumindest in dessen oberen Hälfte gewählt werden kann.

Der genannte Frequenzsteuerbaustein besitzt in Weiterbildung der Erfindung vorteilhafterweise einen Frequenzbeschleunigerbaustein, der die Flackerlichtfrequenz kontinuierlich oder mehrstufig, insbesondere in zumindest mehr als drei Stufen, vorzugsweise mehr als zehn Stufen von der genannten Anfangsfrequenz auf die Zielfrequenz erhöht. Dies erlaubt es, die Flackerlichtfrequenz ausreichend langsam von der Anfangsfrequenz zur Zielfrequenz zu führen, d.h. die Frequenzbeschleunigung wird nicht schlagartig durchgeführt, sondern Schritt für Schritt bzw. kontinuierlich, um den Probanden im Beschleunigungsprozess mitzunehmen.
Alternativ oder zusätzlich kann auch ein manuell zu betätigender Eingabebaustein vorzugsweise in Form eines Tipp-Schalters vorgesehen sein, um eine manuelle Auslösung des Flackerlichts und/oder Steuerung der Flackerlichtfrequenz bzw. die Ein-/Ausphasen des Flackerlichts zu ermöglichen.

Vorteilhafterweise kann die Dauer eines Beschleunigungsdurchlaufs variabel eingestellt werden, wobei vorteilhafterweise die Zeitspanne, die zur Erhöhung der Flackerlichtfrequenz von deren Ausgangsfrequenz zur Zielfrequenz benötigt wird, vorzugsweise im Bereich von 1 min bis 1 h gewählt wird. Um einerseits den Probanden im Beschleunigungsprozess sicher mitzunehmen, andererseits jedoch eine kurze, effiziente Behandlungszeit zu erzielen, sieht der einstellbare Timer für den Beschleunigungsdurchlauf eine Zeitspanne von vorzugsweise mehr als 5 min, ggf. auch mehr als 10 min, jedoch meist weniger als 30 min vor.

Um eine intensive Therapiewirkung zu erzielen, kann es vorteilhaft sein, wenn die Steuervorrichtung mehrere solche Beschleunigungsdurchläufe hintereinander ausführt, ggf. durch Ruhepausen voneinander unterbrochen. Nach einer vorteilhaften Weiterbildung der Erfindung kann das Steuergerät zwei bis drei Beschleunigungsdurchläufe von vorteilhafterweise insgesamt 20 bis 40 min Dauer vorsehen.

Die Lichtstärke der Dauerlichtquelle wie auch die Lichtstärke der Flackerlichtquelle kann grundsätzlich verschieden gewählt werden. In Weiterbildung der Erfindung ist hierbei vorgesehen, dass die Leuchtdichte des Dauerlichts im Bereich des Therapieplatzes variiert werden kann, beispielsweise durch Variieren des von der Dauerlichtquelle abgegebenen Lichtstroms. Hierzu kann der Dauerlichtquelle ein Dimmer zugeordnet sein.

In Weiterbildung der Erfindung sind der Dauerlichtquelle Einstellmittel zur variablen Einstellung der Leuchtdichte des Dauerlichts im Bereich des Therapieplatzes zugeordnet, die von der Steuervorrichtung in Abhängigkeit des Betriebszustandes der Flackerlichtquelle angesteuert werden. Insbesondere kann die Steuervorrichtung einen Leuchtdichte-Steuerbaustein besitzen, der die genannte Leuchtdichte des Dauerlichts in Abhängigkeit der Flackerlichtfrequenz ansteuert derart, dass die Leuchtdichte des Dauerlichts zu Anfang des Beschleunigungsdurchlaufs des Flackerlichts niedriger ist als am Ende des genannten Beschleunigungsdurchlaufs. Insbesondere erreicht die Leuchtdichte des Dauerlichts ihr Maximum erst dann, wenn die Flackerlichtquelle mit ihrer Frequenz in den Bereich der optischen Fusion gefahren ist, in dem die Lichtpulse des Flackerlichts in der Wahrnehmung des Probanden zu einem Dauer- bzw. Konstantlicht verschmelzen.

Alternativ oder zusätzlich kann die Leuchtdichte des Dauerlichts auch unabhängig vom Betriebszustand der Flackerlichtquelle und/oder umgekehrt der Betriebszustand der Flackerlichtquelle unabhängig von der Leuchtdichte des Dauerlichts gesteuert werden. Hierzu kann beispielsweise ein manueller Betätiger bzw. Regler vorgesehen sein. Ebenso kann ggf. das Maximum des Dauerlichts schon vor dem Erreichen der Fusionsgrenze des Flackerlichts vorgesehen werden.

Die Steuervorrichtung kann hierbei grundsätzlich ein stufenloses, stetiges Hochfahren oder auch ein gestuftes Hochfahren der Leuchtdichte des Dauerlichts vorsehen. Vorteilhafterweise wird ein von einem konstanten Anstieg abweichender, zum Ende des Beschleunigungszyklus hin progressiv zunehmender Anstieg der Leuchtdichte des Dauerlichts vorgesehen, um das genannte Licht am Ende des Tunnels intensiver zu simulieren.

Das Dauerlicht und/oder das Flackerlicht können in ihrer Leuchtdichte bzw. ihrer Lichtstärke unterschiedlich ausgebildet sein. Beispielsweise kann jeweils ein Lichtstrom von 500-1500, vorzugsweise 700-900 Lumen und/oder eine Beleuchtungsstärke von 2000-3000 lx und/oder eine Lichtstärke von 100-300 cd, vorzugsweise 200-250 cd vorgesehen sein.

In vorteilhafter Weiterbildung der Erfindung besitzen die Dauerlichtquellen bzw. das davon abgestrahlte Dauerlicht eine andere Farbtemperatur als die Flackerlichtquelle bzw. das davon abgegebene Flackerlicht. Insbesondere von Vorteil ist es hierbei, wenn die zumindest eine Dauerlichtquelle wärmeres Licht abgibt als die zumindest eine Flackerlichtquelle. Hierdurch erhöht sich der kombinatorische Synergieeffekt der unterschiedlich arbeitenden Lichtquellen. Durch kälteres Licht werde die Lichtimpulse des Flackerlichts intensiver, härter wahrgenommen, während das wärmere Dauerlicht die eigentliche Lichterfahrung der Todesnähe bzw. Erfahrungsgrenze besser simuliert. Die konkret zu wählenden Farbtemperaturen können hierbei je nach Behandlungstypus und Proband variiert werden, wobei vorteilhafterweise die Dauerlichtquelle Warmlicht und die Flackerlichtquelle Kaltlicht abgibt. Eine vorteilhafte Ausführung der Erfindung kann hierbei darin bestehen, dass das Dauerlicht der Dauerlichtquelle eine Farbtemperatur im Bereich von 1500 bis 3500 K, vorzugsweise 2000 bis 3000 K, und die Flackerlichtquelle bzw. das davon abgegebene Flackerlicht eine Farbtemperatur von etwa 4000 bis 10000 K, vorzugsweise 5000 bis 8000 K besitzt. Beispielsweise können als Dauerlichtquelle ein Halogenstrahler und als Flackerlichtquelle LEDs Verwendung finden.

Die Erfindung wird nachfolgend anhand eines bevorzugten Ausführungsbeispiels und zugehöriger Zeichnungen näher erläutert. In den Zeichnungen zeigen:
- Fig. 1:: eine schematische Darstellung eines Lichtbehandlungsgeräts in der Bauform eines lampenartigen Einzelgeräts nach einer möglichen vorteilhaften Ausführung der Erfindung, gemäß der mehrere Dauerlichtquellen in Form von Halogenstrahlern mit mehreren Flackerlichtquellen in Form von LEDs kombiniert sind, um auf einem Therapieplatz Flackerlicht mit Dauerlicht zu überlagern,
- Fig. 2:: eine Frontansicht des Lichtbehandlungsgeräts aus Fig. 1, und
- Fig. 3:: eine Frontansicht des Lichtbehandlungsgeräts nach einer weiteren Ausführung, und
- Fig. 4:: ein Ablaufdiagramm zur Verdeutlichung der Variation der Flackerlichtfrequenz während mehrerer aufeinander folgender Beschleunigungsdurchläufe und der darauf abgestimmten Veränderung der Leuchtstärke der Dauerlichtquelle nach einer möglichen, vorteilhaften Ausführung der Erfindung, wobei die durchgezogene Linie die Leuchtstärke der Dauerlichtquelle in Lx und die strichpunktierte Linie die Frequenz der Flackerlichtquelle in Hz angibt.

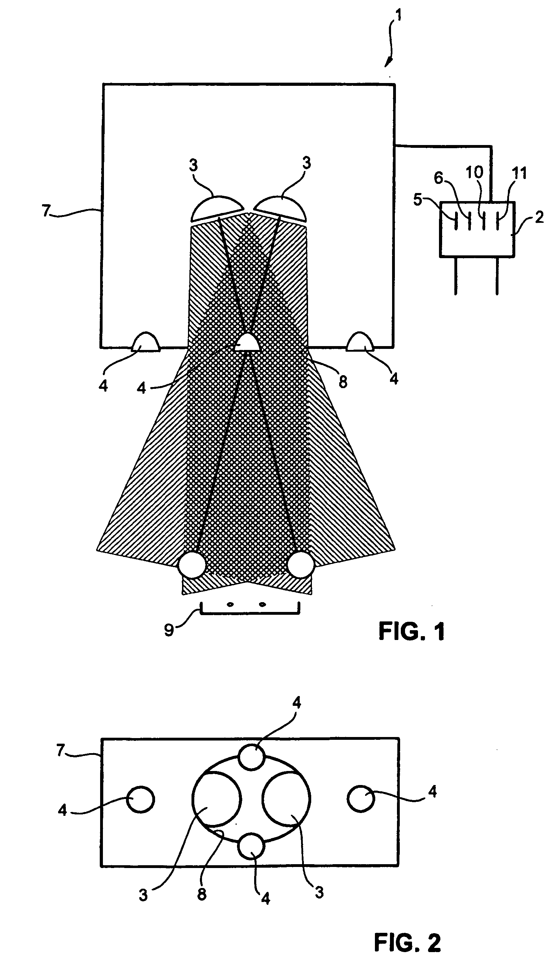

Die in Fig. 1 beispielhaft dargestellte Ausführung der Erfindung zeigt eine Lichtabgabevorrichtung 1, die als lampenartiges Einzelgerät ausgebildet ist. Es versteht sich jedoch, dass die verschiedenen Lichtquellen nicht in einem eine Einheit bildenden Gerät zusammengefasst oder gar in ein Einzelgehäuse eingebaut sein müssen - auch wenn dies eine vorteilhafte Ausführung darstellt -, sondern auch als Rauminstallation, die eine räumlich flexible Platzierung der einzelnen Lichtquellen erlaubt, oder auch als brillenartig tragbares Mobilgerät ausgebildet sein kann.

In der in den Figuren 1 und 2 gezeichneten Ausführung umfasst hierbei die Lichtabgabevorrichtung 1 einen Lichtquellenträger 7, der ein Gehäuse bilden kann und/oder nach Art einer Blende ausgebildet sein kann. In der gezeichneten Ausführungsform sind hierbei hinter einer Blendenöffnung 8 zwei Dauerlichtquellen 3 in Form von Halogenstrahlern angeordnet, deren Lichtkegel durch die Blendenöffnung 8 und/oder durch eine nicht eigens gezeichnete optische Einrichtung wie beispielsweise einen Reflektor und/oder eine Linse auf einen Therapieplatz 9 gerichtet wird, so dass der von den Dauerlichtquellen 3 abgegebene Lichtkegel auf die Augen des Probanden trifft.

Weiterhin trägt der genannte Lichtquellenträger 7 mehrere Flackerlichtquellen 4, wobei in der gezeichneten Ausführungsform vier LEDs als Flackerlichtquellen 4 vorgesehen, die symmetrisch bezüglich der Anordnung der Dauerlichtquellen 3 bzw. der Blendenöffnung 8 angeordnet sind. In der gezeichneten Ausführungsform sind die Flackerlichtquellen 4 dabei außen herum um den aus dem Lichtquellenträger 7 austretenden Lichtkegel der Dauerlichtquellen 3 angeordnet, so dass die Dauerlichtquelle 3 sozusagen aus dem Zentrum der Flackerlichtquellen hervortritt.

Auch die Lichtkegel der Flackerlichtquellen sind auf die Augenposition des auf dem Therapieplatz 9 befindlichen Probanden gerichtet.

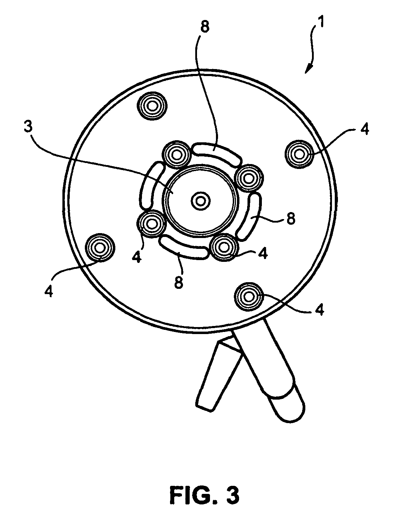

Wie Fig. 3 zeigt, kann die Lichtabgabevorrichtung 1 auch nur eine Dauerlichtquelle 3 besitzen, die gemäß Fig. 3 zentral positioniert und von insgesamt acht Flackerlichtquellen 4, die symetisch verteilt auf zwei Ringen angeordnet sind, umgeben ist.

Die Lichtquellen 3 und 4 werden von einer Steuervorrichtung 2 her angesteuert, die grundsätzlich verschieden aufgebaut sein kann. In der gezeichneten Ausführungsform umfasst sie einen Dauerlicht-Steuerungsbaustein 10 sowie einen Flackerlicht-Steuerungsbaustein 11, die den Betrieb der Dauerlichtquellen 3 bzw. der Flackerlichtquellen 4 hinsichtlich abgegebener Lichtstärke und Pulsung steuern.

Der Flackerlicht-Steuerungsbaustein 11 umfasst hierbei einen Frequenzsteuerbaustein 5, mittels dessen die Frequenz des Flackerlichts variiert wird. Vorteilhafterweise kann der genannte Frequenzsteuerbaustein 5 ein Impulsweitensteuermodul umfassen, um auch die Impulsweite der Lichtimpulse des Flackerlichts zu variieren, so dass nicht nur die Frequenz der Lichtimpulse, sondern auch das Verhältnis der Dauer eines Lichtimpulses zu einer darauf folgenden bzw. vorhergehenden Nicht-Strahlzeit variiert werden kann.

Der genannte Dauerlicht-Steuerungsbaustein 10 kann insbesondere einen Leuchtdichte-Steuerbaustein umfassen, um die Leuchtdichte des Dauerlichts im Bereich des Therapieplatzes 9 variieren zu können, was beispielsweise in einfacher Weise durch einen Leuchtstärkeregler bewerkstelligt werden kann.

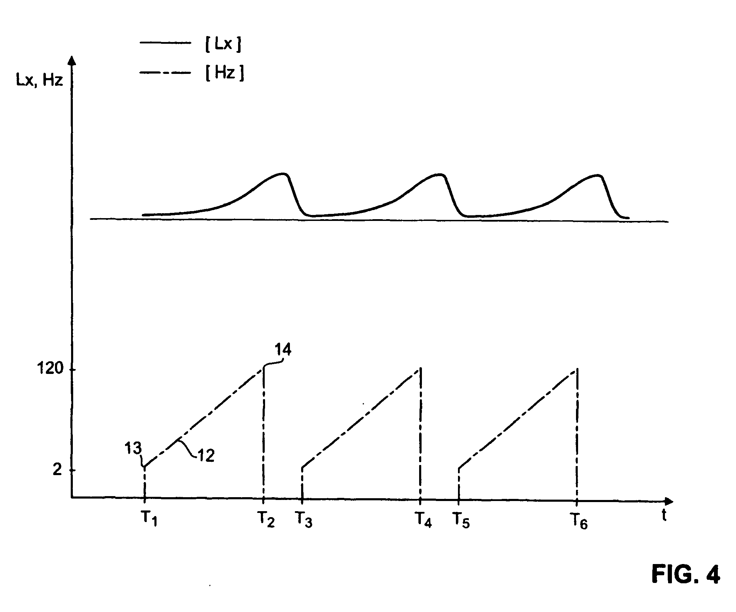

Fig. 4 zeigt beispielhaft einen möglichen Betriebszyklus der Vorrichtung aus den Figuren 1 und 2. Wie Fig. 4 zeigt, wird die - strichpunktiert dargestellte - Flackerlichtfrequenz 12 in mehreren aufeinander folgenden Beschleunigungszyklen T₁ bis T₂, T₃ bis T₄ und T₅ bis T₆ von einer Ausgangsfrequenz 13 kontinuierlich auf eine Zielfrequenz 14 erhöht, wobei die genannte Zielfrequenz 14 vorteilhafterweise knapp oberhalb der optischen Fusionsgrenze liegt. Die Zeitspannen T₁ bis T₂, T₃ bis T₄ und T₅ bis T₆ können vorteilhafterweise im Bereich von einigen Minuten liegen, beispielsweise zwischen 5 und 10 Minuten betragen. In der gezeichneten Ausführung wird dabei die Flackerlichtfrequenz 12 mit konstanter Steigung von zunächst 2 Hz auf 120 Hz erhöht. Zwischen den einzelnen Beschleunigungszyklen sind Entspannungspausen vorgesehen, deren Länge unterschiedlich bemessen sein kann.

Zeitlich abgestimmt auf die Variation der Flackerlichtfrequenz wird auch die Leuchtstärke der Dauerlichtquellen 3 variiert. In der in Fig. 4 gezeichneten, beispielhaften Ausführung wird hierbei die Lichtstärke der Dauerlichtquellen 3 während eines Beschleunigungszyklus T₁ bis T₂ zunächst nur sanft ansteigend erhöht und erst gegen Ende des Beschleunigungszyklus stärker erhöht, so dass die Leuchtstärke der Dauerlichtquellen 3 ihr Maximum erst am Ende bzw. kurz nach dem Erreichen der Zielfrequenz 14 erreicht wird, um das eingangs erwähnte Licht am Ende des Tunnels zu simulieren. Wie Fig. 4 zeigt, können mehrere Beschleunigungszyklen mit entsprechender Anpassung der Dauerlichtquellen abgearbeitet werden.

## Patentansprüche

1. Lichtbehandlungsvorrichtung zur Simulation einer psychophysischen Grenzerfahrung, mit einer Lichtabgabevorrichtung (1) zur Abgabe von über das menschliche Auge aufnehmbaren Lichts, sowie einer Steuervorrichtung (2) zur Steuerung der Lichtabgabevorrichtung (1), wobei die Lichtabgabevorrichtung (2) zumindest eine Flackerlichtquelle (4), umfasst, **dadurch gekennzeichnet, dass** die Lichtabgabevorrichtung (1) zumindest eine Dauerlichtquelle (3) aufweist, wobei das Flackerlicht der Flackerlichtquelle dem Dauerlicht der Dauerlichtquelle (3) überlagerbar ist, und wobei die Steuervorrichtung (2) einen Frequenzsteuerbaustein (5), der in zumindest einem Beschleunigungsdurchlauf die Frequenz der Flackerlichtquelle (4) von einer Ausgangsfrequenz auf eine Zielfrequenz erhöht oder erniedrigt, aufweist.

2. Lichtbehandlungsvorrichtung nach dem vorhergehenden Anspruch, wobei die Zielfrequenz mindestens doppelt so hoch wie die Ausgangsfrequenz gewählt ist.

3. Lichtbehandlungsvorrichtung nach dem vorhergehenden Anspruch, wobei die Zielfrequenz im Bereich der optischen Fusionsgrenze, vorzugsweise oberhalb der optischen Fusionsgrenze, gewählt ist.

4. Lichtbehandlungsvorrichtung nach einem der vorhergehenden Ansprüche, wobei die Zielfrequenz mindestens das Fünffache, vorzugsweise mehr als das Zehnfache, der Ausgangsfrequenz beträgt.

5. Lichtbehandlungsvorrichtung nach einem der vorhergehenden Ansprüche, wobei der Frequenzsteuerbaustein (5) einen variablen Frequenzbereich von 0 Hz bis 1,5 kHz, verzugsweise 2 Hz bis 150 Hz besitzt und die Flackerlichtfrequenz über eine Spanne von zumindest 20 Hz variiert.

6. Lichtbehandlungsvorrichtung nach dem vorhergehenden Anspruch, wobei dem Frequenzsteuerbaustein (5) Einstellmittel zur variablen Einstellung der Anfangsfrequenz und/oder zur variablen Einstellung der Zielfrequenz zugeordnet sind.

7. Lichtbehandlungsvorrichtung nach einem der vorhergehenden Ansprüche, wobei der Frequenzsteuerbaustein (5) einen Frequenzbeschleuniger, der die Flackerlichtfrequenz kontinuierlich oder mehrstufig, vorzugsweise in mehr als drei Stufen, von der genannten Ausgangsfrequenz auf die genannte Zielfrequenz erhöht, aufweist.

8. Lichtbehandlungsvorrichtung nach einem der vorhergehenden Ansprüche, wobei die Steuervorrichtung (2) einen Timer (6) besitzt, der einen Beschleunigungsdurchlauf über eine Zeitspanne von zumindest 1 min, vorzugsweise mehr als 5 min, insbesondere im Bereich von 5 bis 30 min, vorsieht.

9. Lichtbehandlungsvorrichtung nach einem der vorhergehenden Ansprüche, wobei der Dauerlichtquelle (3) Einstellmittel zur variablen Einstellung der Leuchtdichte des Dauerlichts im Therapieplatz zugeordnet sind, die von der Steuervorrichtung (2) in Abhängigkeit des Betriebszustands, vorzugsweise in Abhängigkeit der Flackerlichtfrequenz, der Flackerlichtquelle (4) ansteuerbar sind, insbesondere derart, dass die Leuchtdichte des Dauerlichts bei Betrieb der Flackerlichtquelle (4) mit der Ausgangsfrequenz niedriger ist als bei Betrieb der Flackerlichtquelle (4) im Bereich der Zielfrequenz.

10. Lichtbehandlungsvorrichtung nach dem vorhergehenden Anspruch, wobei die Einstellmittel derart ausgebildet sind, dass die Leuchtdichte des von der Dauerlichtquelle (3) abgegebenen Dauerlichts ihr Maximum erst etwa bei Erreichen der Zielfrequenz der Flackerlichtquelle (4) erreicht.

11. Lichtbehandlungsvorrichtung nach einem der vorhergehenden Ansprüche, wobei die Dauerlichtquelle (3) eine wärmere Farbtemperatur als die Flackerlichtquelle (4) besitzt.

12. Lichtbehandlungsvorrichtung nach dem vorhergehenden Anspruch, wobei das Dauerlicht der Dauerlichtquelle (3) eine Farbtemperatur im Bereich von 1500 bis 3500 K, vorzugsweise 2000 bis 3000 K, und die Flackerlichtquelle eine Farbtemperatur von 4000 bis 10000 K, vorzugsweise 5000 bis 8000 K besitzt.

13. Lichttherapievorrichtung nach einem der vorhergehenden Ansprüche, wobei die maximale Leuchtdichte des Dauerlichts am Therapieplatz mindestens doppelt so groß ist wie die Leuchtdichte des Flackerlichts der Flackerlichtquelle (4).

## Claims

1. A light treatment apparatus for simulating a psychophysical borderline experience comprising a light emitting apparatus (1) for emitting light perceivable by the human eye as well as a control apparatus (2) for controlling the light emission apparatus (1), wherein the light emitting apparatus (1) includes at least one flickering light source (4),
**characterised in that**
the light emitting apparatus (1) has at least one constant light source (3), with the flickering light of the flickering light source being able to be superimposed on the constant light of the constant light source (3), and with the control apparatus (2) having a frequency control module (5) which increases or lowers the frequency of the flickering light source (4) from a starting frequency to a target frequency in at least one acceleration passage.

2. A light treatment apparatus in accordance with the preceding claim, wherein the target frequency is selected to be at least twice as high as the starting frequency.

3. A light treatment apparatus in accordance with the preceding claim, wherein the target frequency is selected in the range of the optical fusion limit, preferably above the optical fusion limit.

4. A light treatment apparatus in accordance with one of the preceding claims, wherein the target frequency amounts to at least five times, preferably more than ten times, the starting frequency.

5. A light treatment apparatus in accordance with one of the preceding claims, wherein the frequency control module (5) has a variable frequency range from 0 Hz to 1.5 kHz, preferably 2 Hz to 150 Hz, and the flickering light frequency varies over a range of at least 20 Hz.

6. A light treatment apparatus in accordance with the preceding claim, wherein adjustment means for the variable setting of the starting frequency and/or for the variable setting of the target frequency are associated with the frequency control module (5).

7. A light treatment apparatus in accordance with one of the preceding claims, wherein the frequency control module (5) has a frequency accelerator which increases the flickering light frequency continuously or in multiple stages, preferably in more than three stages, from the named starting frequency to the named target frequency.

8. A light treatment apparatus in accordance with one of the preceding claims, wherein the control apparatus (2) has a timer (6) which provides an acceleration passage over a time span of at least 1 min, preferably more than 5 min, in particular in the range from 5 to 30 min.

9. A light treatment apparatus in accordance with one of the preceding claims, wherein adjustment means for the variable setting of the luminous density of the permanent light in the treatment area are associated with the constant light source (3) and can be controlled by the control apparatus (2) in dependence on the operating state, preferably in dependence on the flickering light frequency, of the flickering light source (4), in particular such that the luminous density of the constant light is lower on operation of the flickering light source (4) at the starting frequency than on operating the flickering light source (4) in the range of the target frequency (4).

10. A light treatment apparatus in accordance with the preceding claim, wherein the adjustment means are designed such that the luminous density of the constant light emitted by the constant light source (3) only reaches its maximum on approximately reaching the target frequency of the flickering light source (4).

11. A light treatment apparatus in accordance with one of the preceding claims, wherein the constant light source (3) has a warmer colour temperature than the flickering light source (4).

12. A light treatment apparatus in accordance with the preceding claim, wherein the constant light of the constant light source (3) has a colour temperature in the range from 1500 to 3500 K, preferably 2000 to 3000 K, and the flickering light source has a colour temperature from 4000 to 10,000 K, preferably 5000 to 8000 K.

13. A light treatment apparatus in accordance with one of the preceding claims, wherein the maximum luminous density of the constant light at the treatment area is at least twice as large as the luminous density of the flickering light of the flickering light source (4).

## Revendications

1. Dispositif de photothérapie destiné à la simulation d'une expérience psychophysique des limites, avec un dispositif d'émission de lumière (1) destiné à l'émission de lumière captable par l'oeil humain, ainsi qu'un dispositif de commande (2) destiné à la commande du dispositif d'émission de lumière (1), le dispositif d'émission de lumière (1) présentant au moins une source de lumière vacillante, **caractérisé en ce que** le dispositif d'émission de lumière (1) présentant au moins une source de lumière continue (3), la lumière vacillante de la source de lumière vacillante étant superposable à la lumière continue de la source de lumière continue (3), et le dispositif de commande (2) présentant un module de commande de fréquence (5), qui, en au moins un cycle d'accélération augmente ou réduit la fréquence de la source de lumière vacillante (4) d'une fréquence initiale à une fréquence-cible.

2. Dispositif de photothérapie selon la revendication précédente, la fréquence-cible étant sélectionnée au moins au double du niveau de la fréquence initiale.

3. Dispositif de photothérapie selon la revendication précédente, la fréquence-cible étant sélectionnée dans la zone de la limite de fusion optique, de préférence au-dessus de la limite de fusion optique.

4. Dispositif de photothérapie selon une quelconque des revendications précédentes, la fréquence-cible s'élevant au moins au quintuple, de préférence à plus du décuple, de la fréquence initiale.

5. Dispositif de photothérapie selon une quelconque des revendications précédentes, le module de commande de fréquence (5) possédant une gamme des fréquences variable de 0 Hz à 1,5 kHz, de préférence de 2 Hz à 150 Hz et la fréquence de lumière vacillante variant sur une étendue d'au moins 20 Hz.

6. Dispositif de photothérapie selon la revendication précédente, des moyens de réglage étant affectés au module de commande de fréquence (5) pour le réglage variable de la fréquence initiale et/ou pour le réglage variable de la fréquence-cible.

7. Dispositif de photothérapie selon une quelconque des revendications précédentes, le module de commande de fréquence (5) présentant un accélérateur de fréquence qui augmente la fréquence de lumière vacillante en continu ou en plusieurs degrés, de préférence en plus de trois degrés, de ladite fréquence initiale à ladite fréquence-cible.

8. Dispositif de photothérapie selon une quelconque des revendications précédentes, le dispositif de commande (2) possédant une horloge à minuterie (6) qui prévoit un cycle d'accélération sur un écart de temps d'au moins 1 mn, de préférence de plus de 5 mn, notamment dans la zone de 5 à 30 mn.

9. Dispositif de photothérapie selon une quelconque des revendications précédentes, des moyens de réglage étant affectés à la source de lumière continue (3) pour le réglage variable de la densité lumineuse de la lumière continue à l'emplacement de thérapie, qui sont amorçables à partir du dispositif de commande (2) en fonction de l'état de service, de préférence en fonction de la fréquence de lumière vacillante de la source de lumière vacillante (4), notamment de sorte que la densité lumineuse de la lumière continue est inférieure, au fonctionnement de la source de lumière vacillante (4) avec la fréquence initiale, à celle au fonctionnement de la source de lumière vacillante (4) dans la zone de la fréquence-cible.

10. Dispositif de photothérapie selon la revendication précédente, les moyens de réglage étant constitués de sorte que la densité lumineuse de la lumière émise par la source de lumière continue (3) atteint son maximum seulement environ à l'arrivée à la fréquence-cible de la source de lumière vacillante (4).

11. Dispositif de photothérapie selon une quelconque des revendications précédentes, la source de lumière continue (3) possédant une température de couleur plus chaude que la source de lumière vacillante (4).

12. Dispositif de photothérapie selon la revendication précédente, la lumière continue de la source de lumière continue (3) possédant une température de couleur dans la zone de 1500 à 3500 K, de préférence de 2000 à 3000 K, et la source de lumière vacillante possédant une température de couleur de 4000 à 10000 K, de préférence de 5000 à 8000 K.

13. Dispositif de photothérapie selon une quelconque des revendications précédentes, la densité lumineuse maximale de la lumière continue à l'emplacement de thérapie étant au moins deux fois supérieure à la densité lumineuse de la lumière vacillante de la source de lumière vacillante (4).
